(19) Europäisches Patentamt
European Patent Office
Office européen des brevets

(11) EP 1 977 732 B1

(12) **FASCICULE DE BREVET EUROPEEN**

(45) Date de publication et mention
de la délivrance du brevet:
**07.09.2011 Bulletin 2011/36**

(51) Int Cl.:
***A61K 8/60*** *(2006.01)* ***A61K 8/39*** *(2006.01)*
***A61K 8/81*** *(2006.01)* ***A61Q 5/12*** *(2006.01)*
***A61Q 5/02*** *(2006.01)*

(21) Numéro de dépôt: **08152661.8**

(22) Date de dépôt: **12.03.2008**

(54) **Compositions cosmétiques contenant au moins un tensioactif non ionique particulier et au moins un copolymère vinylamide/vinylamine**

Kosmetische Zusammensetzung enthaltend mindestens eine spezifische nicht-ionische Tenside und ein Vinylamid-Vinylamin Copolymer

Cosmetic compositions comprising at least one specific non-ionic surfactant and at least one vinylamide/ vinylamine copolymer

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **02.04.2007 FR 0754210**

(43) Date de publication de la demande:
**08.10.2008 Bulletin 2008/41**

(73) Titulaire: **L'Oréal**
**75008 Paris (FR)**

(72) Inventeurs:
- **Giroud, Franck**
**73390, Chamoux sur Gelon (FR)**
- **Paul, Laurence**
**95320, SAINT LEU LA FORET (FR)**
- **Samain, Henri**
**91570, BIEVRES (FR)**

(74) Mandataire: **Le Blainvaux Bellegarde, Françoise et al**
**L'Oréal**
**D.I.P.I.**
**25-29 Quai Aulagnier**
**92600 Asnières (FR)**

(56) Documents cités:
**WO-A-2007/003784** **DE-A1- 19 540 853**
**DE-A1-102005 014 293** **US-A- 4 713 236**
**US-A- 5 632 977** **US-A1- 2006 286 057**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a trait à des compositions comprenant au moins un tensioactif non ionique particulier et au moins un copolymère vinylamide/vinylamine.

Il est connu d'employer des polymères dans le domaine cosmétique, et notamment en capillaire dans les produits non rincés, par exemple pour apporter de la tenue ou du coiffant à la chevelure.

Dans le domaine des compositions capillaires dites "rincées", telles que les shampooings ou après-shampooings, on utilise aussi des polymères cationiques synthétiques, solubles dans l'eau, qui sont connus pour apporter une bonne cosmétique aux cheveux; toutefois, ces polymères n'apportent aucun effet de mise en forme des cheveux. Il en est de même avec les polymères dérivés naturels cationiques tels les gommes de guar modifiées qui apportent également un caractère cosmétique sans permettre une mise en forme. Dans le domaine des compositions rincées, les polymères n'apportent pas suffisamment de coiffant associée à une cosmétique acceptable.

La présente invention a pour but de proposer des compositions cosmétiques comprenant des polymères capables d'apporter un réel effet coiffant tout en ayant de bonnes propriétés cosmétiques.

Le document W09603969 décrit des compositions capillaires fixantes contenant des polyvinylformamides. Cependant, ces polymères ne permettent pas d'obtenir une tenue durable.
Les demandes US2006/0286057, W02007/03784 et DE10 2005 014293 divulguent des compositions comprenant des copolymères vinylamine/vinylamide mais ne contenant pas de tensioactif non ioniques.

Après de nombreuses recherches, la demanderesse a mis en évidence que l'utilisation de l'association de copolymères vinylformamide/vinylformamine tels que définis ci-après et d'au moins un tensioactif non ionique de type alkylpolyglycoside, pouvait permettre la réalisation de compositions notamment rincées ayant de réelles propriétés de maintien durable et de bonnes propriétés cosmétiques.

Les compositions cosmétiques selon l'invention sont caractérisées par le fait qu'elles comprennent dans un milieu cosmétiquement acceptable :

- un ou plusieurs tensioactifs non ioniques choisis parmi les alkylpolyglycosides et
- un ou plusieurs copolymères vinylformamide/vinylformamine comprenant :

de 10 à 90% en moles de motifs de formule suivante A :

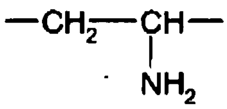
(A)

et de 90 à 10 % en moles de motifs de formule suivante B:

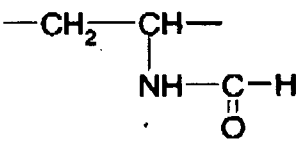
(B)

De manière surprenante, les compositions selon invention possèdent des propriétés cosmétiques intéressantes, par exemple lors de l'application dans une formulation de type shampooing; on a en effet constaté que les cheveux présentent de bonnes propriétés cosmétiques telles que le démêlage, le lissage, la douceur et la brillance, les compositions selon l'invention permettent d'obtenir, une fois la chevelure séchée, une mise en forme des cheveux et une tenue cette mise en forme particulièrement intéressante.

L'invention a également pour objet l'utilisation en cosmétique des compositions ci-dessus pour le nettoyage et /ou le démaquillage et/ou le conditionnement des matières kératiniques telles que les cheveux et la peau. L'invention a encore pour objet une utilisation de la composition selon l'invention comme shampoing des matières kératiniques.

Tensioactif(s) non ionique(s) :

En ce qui concerne les alkypolyglycosides, ces composés sont bien connus et peuvent être plus particulièrement représenté par la formule générale suivante :

$$R_1O\text{-}(R_2O)_t(G)_v \qquad (I)$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (I) dans laquelle $R_1$ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (I), peut aller de 1 à 15, de préférence de 1 à 4. Le degré moyen de polymérisation est plus particulièrement compris entre 1 et 2 et encore plus préférentiellement de 1,1 à 1,5.
Les liaisons glycosidiques entre les motifs sucre sont de type 1-6 ou 1-4 et de préférence 1-4.

Des composés de formule (I) sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.
On peut également utiliser par exemple, l'Alkyl en C8/C16 polyglucoside 1,4 en solution aqueuse à 53% commercialisé par COGNIS sous la référence PLANTACARE® 818 UP.

Le ou les tensioactifs non ioniques choisis parmi les alkylpolyglycosides sont de préférence les alkyl C6-C24 polyglucosides et plus particulièrement les alkyl C8-C16 polyglucosides.

Le ou les tensioactifs non ioniques choisis parmi les alkylpolyglycosides sont présents dans les compositions selon l'invention dans des concentrations allant de 0,1 à 40% en poids, de 1 à 30% en poids, plus particulièrement de 5 à 25 % en poids, de préférence de 8 à 20% en poids par rapport au poids total de la composition.

Copolymère vinylformamide/vinylformamine

Les copolymères vinylamine/vinylamide selon l'invention comprennent de préférence de 10 à 60% en moles de motif de formule A et plus particulièrement de 20 à 40% en moles.

Les copolymères vinylamine/vinylamide selon l'invention comprennent de préférence de 30 à 90% en moles de motif de formule B et plus particulièrement de 60 à 80% en moles.

Les copolymères selon l'invention peuvent être obtenus par hydrolyse partielle de polyvinylformamide. L'hydrolyse peut se faire en milieu acide ou basique.

Les copolymères vinylamine/vinylamide selon l'invention peuvent éventuellement comprendre un ou plusieurs motifs monomères additionnels. Dans ce cas, ils représentent moins de 20% en moles du copolymère.

De préférence, les copolymères vinylamine/vinylamide selon l'invention sont constitués uniquement de motifs A et de motifs B.

Le poids moléculaire en poids mesuré par diffraction de la lumière peut varier 10.000 à 30.000.000 g/mole, de préférence de 40.000 à 1.000.000 et plus particulièrement de 100.000 à 500.000 g/mole.

La densité de charge cationique du copolymère vinylamine/vinylamide à pH 5 peut varier de 2 meq/g à 20 meq/g, de préférence de 2,5 à 15 et plus particulièrement de 3,5 à 10 meq/g.

Comme copolymères vinylamine/vinylamide utilisables selon la présente invention, on peut citer les LUPAMIN 9030 et 9010 proposés par la société BASF.

Le ou les copolymères vinylamine/vinylamide sont présents, dans les compositions selon l'invention, dans des proportions allant, de préférence, de 0,01 à 20 % en poids et de préférence de 0,05 à 10 % en poids par rapport au poids total de la composition et plus particulièrement de 0,1 à 5% en poids.

Les compositions selon l'invention peuvent comprendre en outre un ou plusieurs tensioactifs additionnels tels que par exemple des tensioactifs anioniques, amphotères, zwittérioniques et leurs mélanges. Les compositions peuvent également comprendre des tensioactifs non ioniques différents des tensioactifs non ioniques choisis parmi les alkylpolyglycosides et les tensioactifs mono ou polyglycérolés.

Pour ce qui a trait aux tensioactifs amphotères ou zwittérioniques, on peut mentionner sans avoir l'intention

de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives : $R_2$ -$CONHCH_2CH_2$ -$N(R_3)(R_4)(CH_2COO^-)$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-}CONHCH_2CH_2\text{-}N(B)(C)$$

dans laquelle :

B représente -$CH_2CH_2OX'$, C représente -$(CH_2)_z$-Y', avec z = 1 ou 2,
X' désigne le groupement -$CH_2CH_2$-COOH ou un atome d'hydrogène
Y' désigne -COOH ou le radical -$CH_2$-CHOH-$SO_3H$
$R_2'$ désigne un radical alkyle d'un acide $R_9$-COOH présent dans l'huile de coprah ou dans l'huile de lin hydrolysée, un radical alkyle, notamment en$C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylampho-dipropionate, Disodium Capryloamphodipropionate, Lauroamphodipropionic acid, Cocoamphodipropionic acid, disodium cocoamphocarboxy ethyl hydroxypropyl sulfonate.

A titre d'exemple on peut citer le cocoamphodiacetate commercialisé sous la dénomination commerciale MIRANOL® C2M concentré par la société Rhodia Chimie.

Les composition selon l'invention peuvent également contenir des tensioactifs anioniques et plus particulièrement des tensioactifs anioniques carboxyliques.

A titre d'exemple de tensio-actifs anioniques utilisables, seuls ou en mélange, dans le cadre de la présente invention, on peut citer notamment les sels (en particulier sels alcalins, notamment sels de sodium, sels d'ammonium, sels d'amines, sels d'aminoalcools ou sels de magnésium) des composés suivants : les alkylsulfates, alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, alkyléthersulfosuccinates, alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle.

Parmi les tensioactifs anioniques plus particulièrement préférés, on peut également citer des tensioactifs anioniques carboxyliques, comme les acides d'alkyl D-galactoside uroniques et leurs sels ainsi que les acides éthers carboxyliques polyoxyalkylénés et leurs sels, en particulier ceux comportant de 2 à 50 groupements oxyde d'éthylène, et leurs mélanges.

Les tensioactifs anioniques du type acides ou sels d'éthers carboxyliques polyoxyalkylénés sont en particulier ceux qui répondent à la formule (II) suivante :

$$R_1\text{—}(OC_2H_4)_n\text{—}OCH_2COOA \qquad \text{(II)}$$

dans laquelle :

- $R_1$ désigne un groupement alkyle, alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
- A désigne H, ammonium, Na, K, Li, Mg ou un reste monoéthanolamine ou triéthanolamine. On peut également utiliser des mélanges de composés de formule (II) en particulier des mélanges dans lesquels les groupements $R_1$

sont différents.

Des composés de formule (II) sont vendus par exemple par la Société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

Tout particulièrement, on utilise des acides alkyl éther carboxyliques polyoxyalkylénés comme par exemple l'acide lauryl ether carboxylique (4,5 OE) commercialisé par exemple sous la dénomination AKYPO RLM 45 CA de KAO.

En association avec les tensioactifs anioniques carboxyliques, les tensioactifs anioniques convenant plus particulièrement aux compositions selon l'invention sont choisis parmi le lauryléther sulfate de sodium ou d'ammonium, le lauryl sulfate de sodium ou d'ammonium et/ou leurs mélanges.

Si de tels tensioactifs additionnels sont présents, alors la teneur en tensioactif(s) additionnel(s) va de 0,1 à 20% en poids, par rapport au poids total de la composition, plus particulièrement de 1% à 15 % en poids, et encore mieux de 1,5 à 10% en poids par rapport au poids total de la composition.

Les compositions selon l'invention comprennent de préférence une concentration totale en tensioactifs allant de 4 à 50% en poids, plus particulièrement de 8 à 30% en poids par rapport au poids total de la composition.

De préférence, la composition selon l'invention comprend au moins un agent conditionneur additionnel différent du copolymère de vinylamine/vinylamide. Lorsque la composition contient au moins un agent conditionneur, il est généralement choisi parmi les huiles de synthèse telles que les poly-$\alpha$-oléfines, les huiles fluorées, les cires fluorées, les gommes fluorées, les cires non fluorées, les esters gras d'acides carboxyliques, les polymères cationiques, les silicones, les huiles minérales, végétales ou animales, les céramides, les pseudocéramides et leurs mélanges.

Selon un mode de réalisation préféré, le ou les agents conditionneurs sont choisis parmi les polymères cationiques différents des copolymères vinylamine/vinylamide selon l'invention, les silicones, et leurs mélanges. De préférence l'agent conditionneur est choisi parmi les silicones, de préférence non volatiles.

Selon ce mode de réalisation, la teneur en agent conditionneur additionnel dans la composition selon l'invention est comprise de 0,001 % à 25 % en poids, de préférence de 0,005 % à 10 % en poids, et encore plus préférentiellement de 0,01 % à 5 % en poids, du poids total de la composition finale.

Le ou les polymères cationiques sont généralement présents à des concentrations allant de 0,01 à 20 %, de préférence de 0,05 à 10 % et plus particulièrement de 0,1 à 5 % en poids par rapport au poids total de la composition.

A titre de silicones utilisables dans les compositions de la présente invention, on peut notamment citer les silicones volatiles ou non, cycliques ou acycliques, ramifiées ou non, organomodifiées ou non.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Les silicones sont de préférence choisies parmi les polydiméthylsiloxanes non ionique à groupements terminaux triméthylsilyles ou diméthylsilanols (Dimethicone ou dimethiconol selon la nomenclature INCI).

Selon l'invention, toutes les silicones peuvent être utilisées telle quelle ou sous forme de solutions, de dispersions, d'émulsions, de nanoémulsions ou de microémulsions.

Les silicones peuvent être utilisées seules ou en mélange, en une quantité allant de 0,01 à 20 % en poids, de préférence de 0,1 à 5 % en poids par rapport au poids total de la composition.

La composition selon l'invention peut également comprendre des ingrédients couramment utilisés en cosmétique, tels que les vitamines, les parfums, les agents nacrants, les épaississants, les polymères différents des polymères de l'invention, les gélifiants, les oligo-éléments, les adoucissants, les séquestrants, les agents alcalinisants ou acidifiants, les conservateurs, les filtres solaires, les anti-oxydants, les agents anti-chute des cheveux, les agents anti-pelliculaires, les agents anti-gras, les anti-radicaux libres, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, le pH de la composition de la présente invention est choisi dans la gamme allant de 2 à 11 et préférentiellement de 3 à 10 par exemple de 5 à 8.

La composition selon l'invention peut comprendre un propulseur. Le propulseur est choisi notamment parmi les gaz comprimés ou liquéfiés usuellement employés pour la préparation de compositions aérosols et leurs mélanges. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.
Les compositions selon l'invention sont de préférence des compositions détergentes telles que des shampooings, des gels-douche et des bains moussants. Dans ce mode de réalisation de l'invention, les compositions comprennent au moins 4% en poids par rapport au poids total de la composition d'au moins un tensioactif détergent anionique et/ou non ionique.

L'invention a donc encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.
Ainsi, ce procédé selon l'invention permet le maintien de la coiffure, le traitement, le soin et de préférence le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, ou encore sous forme de compositions à rincer, à appliquer avant ou après tout traitement capillaire, notamment une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement au moins un tensioactif cationique, par exemple en une concentration allant généralement de 0,1 à 10% en poids et de préférence de 0,5 à 5% en poids par rapport au poids total de la composition.

La composition suivant l'invention, après application sur les cheveux et cuir chevelu humains peut être rincée ou non rincée après tout traitement. Elle est de préférence rincée. Elle peut se présenter sous toute forme classiquement utilisée dans le domaine concerné et par exemple sous forme de lotion plus ou moins épaissie, de gel, de crème, de spray ou de mousse. Cette composition peut être monophasique ou multiphasique.

Selon un mode de réalisation préféré de l'invention, la composition peut être utilisée comme shampoing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau ou avec une composition aqueuse, l'opération pouvant être répétée une ou plusieurs fois.

Les compositions de l'invention sont illustrées plus en détail dans les exemples suivants.

Les pourcentages sont exprimés en pourcentage en poids de matière active.

EXEMPLES

On a préparé les compositions de shampooing suivantes :

| En %MA | 1 invention | 2 | 3 | 4 | 5 | 6 |
|---|---|---|---|---|---|---|
| Cocoglucoside (Plantacare 818 UP de COGNIS) | 12,5 | 12,5 | 12,5 | 12,5 | | 12,5 |
| Copolymère vinylformamide/vinyl formamine (70/30 en moles) (LUPAMIN 9030 de BASF) | 1 | - | - | | 1 | - |
| Polyvinylformamide (LUPAMIN 9000 de BASF) | - | 1 | | | | - |
| Polyvinylformamine (LUPAMIN 9095 de BASF) | | | 1 | | | |
| Polyéthylèneimine (LUPASOL P e BASF) | | | | 1 | | |
| Oleth-10 (BRIJ 96 de UNIQEMA) | | | | | 12,5 | |
| Conservateur | qs | qs | qs | qs | qs | qs |
| Parfum | qs | qs | qs | qs | qs | qs |
| Agent de pH qs | pH 6,5 | pH 6,5 | pH 6,5 | pH 6,5 | pH 6,5 | pH 6,5 |
| Eau qsp | 100 | 100 | 100 | 100 | 100 | 100 |

Evaluation de la rétention de boucle

1 gramme de chaque formule est appliqué sur une mèche de cheveux naturels caucasiens de 25cm de longueur (l) environ pesant 2.7g.

Les mèches sont malaxées, laissées posées 5 minutes puis rincées.

Les mèches humides sont ensuite enroulées sur un bigoudi (0 =2cm) puis séchées au casque 30 minutes à 70°C.

Les bigoudis sont retirés, et l'on obtient une « anglaise », c'est-à-dire une mèche enroulée en spirale de longueur variable.

Ces mèches sont ensuite suspendues à un crochet, c'est à dire soumises à leur propre poids.

Après suspension la longueur initiale ($l_0$) de la mèche enroulée est mesurée.

Les mèches sont ensuite conditionnées 24 heures dans une boite à gant à hygrométrie et température contrôlées (25 °C / 45 % Humidité Relative).

Après 24 heures de suspension la longueur ($l_t$) des mèches qui se sont détendues sous l'action de leur propre poids est de nouveau mesurée.

Le % de rétention de boucle est calculé par l'équation suivante :

$$\% \text{ de rétention} = \left[\frac{(l - l_t)}{(l - l_0)}\right] * 100$$

l: longueur de la mèche avant enroulement
$l_0$: longueur de la mèche bouclée juste après suspension
$l_t$: longueur de la mèche bouclée après 24 heures de suspension

Plus la valeur obtenue est proche de 100% et plus la mise en forme de la mèche est marquée et durable.

| | **Invention ex 1** | (ex2) | (ex 3) | (ex 4) | (ex 5) | (ex6) |
|---|---|---|---|---|---|---|
| % rétention de boucle | **75%** | 53% | 66% | 61% | 57% | 60% |

Les cheveux traits avec la composition de l'exemple 1 présentent une mise en forme plus durable. De plus, les cheveux traités avec la composition de l'exemple 1 présentent de bonnes propriétés de démêlage et de lissage. Ils sont également doux et brillants.

| En gMA | **7 Invention** | 8 | 9 | 10 | 11 |
|---|---|---|---|---|---|
| Cocoglucoside (Plantacare 818 UP de COGNIS) | 11 | 11 | 11 | 11 | 11 |
| Coco amido propyl bétaine | 2,6 | 2,6 | 2,6 | 2,6 | 2,6 |
| Lauryléther carboxylate de sodium | 1,8 | 1,8 | 1,8 | 1,8 | 1,8 |
| Copolymère vinyl formamide /vinylformamine (70/30 en moles) (LUPAMIN 9030 de BASF) | 1 | - | - | - | - |
| Polyvinylformamide (LUPAMIN 9000 de BASF) | - | 1 | - | - | - |
| Polyvinylformamine (LUPAMIN 9095 de BASF) | - | - | 1 | - | - |
| Polyéthylèneimine (LUPASOL P de BASF) | - | - | - | 1 | - |
| Conservateur | qs | qs | qs | qs | qs |
| Parfum | qs | qs | qs | qs | qs |
| Agent de pH qs | pH 6,5 | pH 6,5 | pH 6,5 | pH 6,5 | pH 6,5 |
| Eau qsp | 100 | 100 | 100 | 100 | 100 |

| | **Invention (ex 7)** | (ex 8) | (ex 9) | (ex 10) | (ex 11) |
|---|---|---|---|---|---|
| % rétention de boucle | **78%** | 57% | 63% | 52% | 50% |

Les cheveux traités avec la composition de l'exemple 1 présentent une mise en forme plus durable.
De plus, les cheveux traités avec la composition de l'exemple 1 présentent de bonnes propriétés de démêlage et de lissage. Ils sont également doux et brillants.

Compositions aérosol selon l'invention

On a préparé les compositions suivantes :

| En gMA | **12** | **13** |
|---|---|---|
| Laureth 5 carboxylic acid | - | 1,8 g |
| Coco amidopropyl betaine | - | 2,6 g |
| Coco glucoside | 12,5 g | 11 g |
| Polyglyceryl-3hydroxylauryléther (Chimexane NF de CHIMEX) | | |
| Copolymère vinylformamide/vinylformamine (70/30 en moles (LUPAMIN 9030 de BASF) | 1 | 1 |
| Conservateur | Qs | Qs |
| Parfum | Qs | Qs |
| Agent de pH qs | pH 6.5 | pH 6.5 |
| Isobutane / propane 1 butane (PROPEL 45 de REPSOL) | 5 g | 5 g |
| Eau qsp | 100 g | 100 g |

## Revendications

1. Composition cosmétique **caractérisée par le fait qu'**elle comprend dans un milieu aqueux cosmétiquement acceptable:

- un ou plusieurs tensioactifs non ioniques choisis parmi les alkylpolyglycosides et
- un ou plusieurs copolymères vinylformamide/vinylformamine comprenant :

de 10 à 90% en moles de motif de formule suivante A:

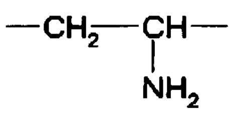

(A)

et de 90 à 10 % en moles de motifs de formule suivante B:

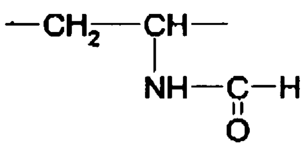

(B)

2. Composition selon la revendication 1, **caractérisé en ce que** les alkylpolyglucosides sont représenté par la formule

générale suivante :

$$R_1O\text{-}(R_2O)_t\ (G)_v \qquad (I)$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un sucre comportant de 5 à 6 atomes de carbone, t désigne une valeur allant de 0 à 10, de préférence 0 à 4, et v désigne une valeur allant de 1 à 15.

**3.** Composition selon la revendication précédente, **caractérisée en ce que** dans la formule (I) $R_1$ désigne un radical alkyle saturé ou insaturé, linéaire ou ramifié comportant de 8 à 18 atomes de carbone, t désigne une valeur égale à 0, G désigne le glucose, le fructose ou le galactose, v est une valeur allant 1 à 4.

**4.** Composition selon l'une des revendications 2 ou 3, **caractérisée en ce que** dans la formule (I), G désigne le glucose et v est une valeur allant de 1,1 à 2.

**5.** Composition selon l'une des revendications précédentes, **caractérisée en ce que** le ou les tensioactifs non ioniques sont présents dans les compositions selon l'invention dans des concentrations allant de 0,1 à 40% en poids, de1 à 30% en poids, de préférence de 5 à 25 % en poids par rapport au poids total de la composition.

**6.** Composition selon l'une des revendications précédentes, **caractérisée en ce que** le ou les copolymères vinylamine/vinylamide comprennent de 10 à 60% en moles de motif de formule A et plus particulièrement de 20 à 40% en moles.

**7.** Composition selon l'une des revendications précédentes, **caractérisée en ce que** le ou les le ou les copolymères vinylamine/vinylamide sont présents dans les compositions selon l'invention dans des concentrations de 0,01 à 20 % en poids et de préférence de 0, 05 à 10 % en poids par rapport au poids total de la composition et plus particulièrement de 0,1 à 5% en poids.

**8.** Composition selon l'une des revendications précédentes, **caractérisée en ce que** la composition comprend en outre un ou plusieurs tensioactifs additionnels.

**9.** Composition selon la revendication précédente, **caractérisée en ce que** la teneur en tensioactif(s) additionnel(s) va de 0,1 à 20% en poids, par rapport au poids total de la composition, plus particulièrement de 1% à 15 % en poids, par rapport au poids total de la composition.

**10.** Composition selon l'une quelconque des revendications précédentes, **caractérisée en ce qu'**elle comprend en outre au moins un agent conditionneur additionnel.

**11.** Composition selon la revendication précédente, **caractérisée en ce que** le ou les agents conditionneurs additionnels sont choisis parmi les poly-α-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges.

**12.** Composition selon l'une quelconque des revendications 10 ou 11, **caractérisé en ce que** le ou les agents conditionneurs additionnels sont choisis parmi les polymères cationiques, les silicones et leurs mélanges.

**13.** Composition selon la revendication précédente, **caractérisé en ce que** l'agent conditionneur additionnel est une silicone.

**14.** Composition selon l'une quelconque des revendications 10 à 12, **caractérisé en ce que** l'agent conditionneur est un polymère cationique.

**15.** Composition selon l'une quelconque des revendications 10 à 14, **caractérisée en ce** les agents conditionneurs représentent de 0,001 % à 25 % en poids, de préférence de 0,005 % à 10 % en poids, par rapport au poids total de la composition.

**16.** Composition selon l'une quelconque des revendications précédentes, **caractérisée par le fait que** la composition

comprend en outre au moins un additif choisi les alcools gras ayant de 12 à 26 atomes de carbone ; les polymères différents des polymères de l'invention; les vitamines, les parfums, les agents nacrants, les épaississants, les gélifiants, les oligo-éléments, les adoucissants, les séquestrants, les agents alcalinisants ou acidifiants, les conservateurs, les filtres solaires, les anti-oxydants, les agents anti-chutes des cheveux, les agents anti-pelliculaires, les agents anti-gras, les agents propulseurs; leurs mélanges.

**17.** Composition selon l'une des revendications précédentes, dans laquelle le milieu cosmétiquement acceptable comprend au moins un constituant choisi parmi l'eau ; les solvants organiques hydrophiles comme les alcools, notamment les monoalcools, linéaires ou ramifiés en C1-C6 et les polyols et les éthers de glycols

**18.** Composition selon l'une des revendications précédentes, se présentant sous la forme d'une composition capillaire, notamment pour le maintien de la coiffure ou la mise en forme des cheveux, par exemple sous forme de shampooings, de gels, de lotions de mise en plis, de lotions pour le brushing, de compositions de fixation et de coiffage telles que les laques ou spray ; d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

**19.** Utilisation d'une composition telle définie dans l'une quelconque des revendications 1 à 18 pour le nettoyage et/ou le démaquillage et/ou le conditionnement des matières kératiniques.

**20.** Utilisation d'une composition telle définie dans l'une quelconque des revendications 1 à 18 comme shampooing des matières kératiniques

**21.** Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 1 à 18 et à faire suivre ou non l'application d'un rinçage après un éventuel temps de pause.

**22.** Procédé de lavage des matières kératiniques en particulier des cheveux, **caractérisé par le fait qu'**il consiste à appliquer sur lesdites matières une composition telle que définie selon l'une quelconque des revendications 1 à 18 puis à effectuer éventuellement un rinçage à l'eau.

## Claims

**1.** Cosmetic composition, **characterized in that** it comprises, in a cosmetically acceptable aqueous medium:

- one or more non-ionic surfactants chosen from alkylpolyglycosides, and
- one or more vinylformamide/vinylamine copolymers comprising:

from 10 to 90 mol% of units of following formula A:

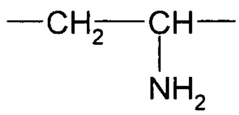

(A)

and from 90 to 10 mol% of units of following formula B:

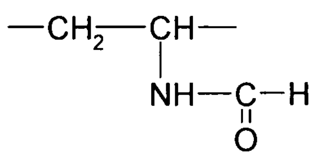

(B)

2. Composition according to Claim 1, **characterized in that** the alkylpolyglycosides are represented by the following general formula:

$$R_1O\text{-}(R_2O)_t(G)_v \qquad (I)$$

in which $R_1$ represents a linear or branched alkyl and/or alkenyl radical comprising approximately from 8 to 24 carbon atoms or an alkylphenyl radical, the linear or branched alkyl radical of which comprises from 8 to 24 carbon atoms, $R_2$ represents an alkylene radical comprising approximately from 2 to 4 carbon atoms, G represents a sugar comprising 5 or 6 carbon atoms, t denotes a value ranging from 0 to 10, preferably from 0 to 4, and v denotes a value ranging from 1 to 15.

3. Composition according to the preceding claim, **characterized in that**, in the formula (I), $R_1$ denotes a saturated or unsaturated and linear or branched alkyl radical comprising from 8 to 18 carbon atoms, t denotes a value equal to 0, G denotes glucose, fructose or galactose, and v is a value ranging from 1 to 4.

4. Composition according to either of Claims 2 and 3, **characterized in that**, in the formula (I), G denotes glucose and v is a value ranging from 1.1 to 2.

5. Composition according to one of the preceding claims, **characterized in that** the non-ionic surfactant or surfactants are present in the compositions according to the invention in concentrations ranging from 0.1 to 40% by weight, from 1 to 30% by weight and preferably from 5 to 25% by weight, with respect to the total weight of the composition.

6. Composition according to one of the preceding claims, **characterized in that** the vinylamine/vinylamide copolymer or copolymers comprise from 10 to 60 mol% of units of formula A and more particularly from 20 to 40 mol%.

7. Composition according to one of the preceding claims, **characterized in that** the vinylamine/vinylamide copolymer or copolymers are present in the compositions according to the invention in concentrations from 0.01 to 20% by weight and preferably from 0.05 to 10% by weight, with respect to the total weight of the composition, more particularly from 0.1 to 5% by weight.

8. Composition according to one of the preceding claims, **characterized in that** the composition additionally comprises one or more additional surfactants.

9. Composition according to the preceding claim, **characterized in that** the content of additional surfactant(s) ranges from 0.1 to 20% by weight, with respect to the total weight of the composition, more particularly from 1 to 15% by weight, with respect to the total weight of the composition.

10. Composition according to any one of the preceding claims, **characterized in that** it additionally comprises at least one additional conditioning agent.

11. Composition according to the preceding claim, **characterized in that** the additional conditioning agent or agents are chosen from poly-$\alpha$-olefins, fluorinated oils, fluorinated waxes, fluorinated gums, esters of carboxylic acids, silicones, cationic polymers, mineral, vegetable or animal oils, ceramides, pseudoceramides and their mixtures.

12. Composition according to either one of Claims 10 and 11, **characterized in that** the additional conditioning agent or agents are chosen from cationic polymers, silicones and their mixtures.

13. Composition according to the preceding claim, **characterized in that** the additional conditioning agent is a silicone.

14. Composition according to any one of Claims 10 to 12, **characterized in that** the conditioning agent is a cationic polymer.

15. Composition according to any one of Claims 10 to 14, **characterized in that** the conditioning agents represent from 0.001% to 25% by weight, preferably from 0.005% to 10% by weight, with respect to the total weight of the composition.

16. Composition according to any one of the preceding claims, **characterized in that** the composition additionally comprises at least one additive chosen from fatty alcohols having from 12 to 26 carbon atoms; polymers other than the polymers of the invention; vitamins, fragrances, pearlescent agents, thickeners, gelling agents, trace elements,

softeners, sequestering agents, basifying or acidifying agents, preservatives, sunscreens, antioxidants, agents for combating hair loss, antidandruff agents, agents for combating fat, propellants; or their mixtures.

**17.** Composition according to one of the preceding claims, in which the cosmetically acceptable medium comprises at least one constituent chosen from water, hydrophilic organic solvents, such as alcohols, in particular linear or branched $C_1$-$C_6$ monoalcohols, and polyols and glycol ethers.

**18.** Composition according to one of the preceding claims which is provided in the form of a hair composition, in particular for the form retention of the hairstyle or the shaping of the hair, for example in the form of shampoos, gels, hair setting lotions, blow drying lotions or fixing and styling compositions, such as lacquers or sprays; of a rinse-out or leave-in conditioner, of permanent waving, hair straightening, dyeing or bleaching compositions, or in the form of rinse-out compositions to be applied before or after a dyeing operation, a bleaching operation, a permanent waving operation or a hair straightening operation or between the two stages of a permanent waving operation or of a hair straightening operation.

**19.** Use of a composition as defined in any one of Claims 1 to 18 for cleaning and/or removing makeup from and/or conditioning keratinous substances.

**20.** Use of a composition as defined in any one of Claims 1 to 18 as shampoo for keratinous substances.

**21.** Cosmetic method for treating keratinous substances, such as the skin of the body or of the face, nails, body hair, head hair and/or eyelashes, **characterized in that** it consists in applying, to the keratinous substances, a cosmetic composition as defined in one of Claims 1 to 18 and in following or not following the application with a rinsing operation after an optional leave-in time.

**22.** Method for washing keratinous substances, in particular the hair, **characterized in that** it consists in applying, to the said substances, a composition as defined according to any one of Claims 1 to 18 and in then optionally rinsing with water.

**Patentansprüche**

**1.** Kosmetische Zusammensetzung, **dadurch gekennzeichnet, dass** sie in einem kosmetisch unbedenklichen wässrigen Medium Folgendes umfasst:

- ein oder mehrere nichtionische Tenside, ausgewählt aus Alkylpolyglykosiden, und
- ein oder mehrere Vinylformamid-Vinylamin-Copolymere, umfassend: 10 bis 90 Mol-% Einheiten der folgenden Formel A:

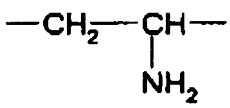

(A)

und 90 bis 10 Mol-% Einheiten der folgenden Formel B:

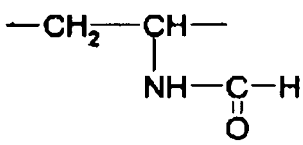

(B)

**2.** Zusammensetzung nach Anspruch 1, **dadurch gekennzeichnet, dass** die Alkylpolyglykoside durch die folgende allgemeine Formel dargestellt werden:

$$R_1O\text{-}(R_2O)_t(G)_v \qquad (I)$$

in der $R_1$ für einen linearen oder verzweigten, etwa 8 bis 24 Kohlenstoffatome umfassenden Alkyl- und/oder Alkenylrest oder einen Alkylphenylrest steht, dessen linearer oder verzweigter Alkylrest 8 bis 24 Kohlenstoffatome umfasst, $R_2$ für einen etwa 2 bis 4 Kohlenstoffatome umfassenden Alkylenrest steht, G für einen 5 bis 6 Kohlenstoffatome umfassenden Zucker steht, t für einen Wert von 0 bis 10, vorzugsweise von 0 bis 4, steht und v für einen Wert von 1 bis 15 steht.

3. Zusammensetzung nach dem vorhergehenden Anspruch, **dadurch gekennzeichnet, dass** in der Formel (I) $R_1$ für einen gesättigten oder ungesättigten, linearen oder verzweigten, 8 bis 18 Kohlenstoffatome umfassenden Alkylrest steht, t für einen Wert gleich 0 steht, G für Glucose, Fructose oder Galactose steht und v für einen Wert von 1 bis 4 steht.

4. Zusammensetzung nach einem der Ansprüche 2 oder 3, **dadurch gekennzeichnet, dass** in der Formel (I) G für Glucose steht und v für einen Wert von 1, 1 bis 2 steht.

5. Zusammensetzung nach einem der vorhergehenden Ansprüche, **dadurch gekennzeichnet, dass** das oder die nichtionischen Tenside in den erfindungsgemäßen Zusammensetzungen in Konzentrationen von 0,1 bis 40 Gew.-%, von 1 bis 30 Gew.-%, vorzugsweise von 5 bis 25 Gew.-%, bezogen auf das Gesamtgewicht der Zusammensetzung, vorhanden sind.

6. Zusammensetzung nach einem der vorhergehenden Ansprüche, **dadurch gekennzeichnet, dass** das oder die Vinylamin-Vinylamid-Copolymere 10 bis 60 Mol-% Einheiten der Formel A und insbesondere von 20 bis 40 Mol-% umfassen.

7. Zusammensetzung nach einem der vorhergehenden Ansprüche, **dadurch gekennzeichnet, dass** das oder die Vinylamin-Vinylamid-Copolymere in den erfindungsgemäßen Zusammensetzungen in Konzentrationen von 0,01 bis 20 Gew.-% und vorzugsweise von 0,05 bis 10 Gew.-% und insbesondere von 0,1 bis 5 Gew.-%, bezogen auf das Gesamtgewicht der Zusammensetzung, vorhanden sind.

8. Zusammensetzung nach einem der vorhergehenden Ansprüche, **dadurch gekennzeichnet, dass** die Zusammensetzung des Weiteren ein oder mehrere zusätzliche Tenside umfasst.

9. Zusammensetzung nach dem vorhergehenden Anspruch, **dadurch gekennzeichnet, dass** der Gehalt an zusätzlichem Tensid/zusätzlichen Tensiden 0,1 bis 20 Gew.-%, bezogen auf das Gesamtgewicht der Zusammensetzung, insbesondere 1 bis 15 Gew.-% bezogen auf das Gesamtgewicht der Zusammensetzung, beträgt.

10. Zusammensetzung nach einem der vorhergehenden Ansprüche, **dadurch gekennzeichnet, dass** sie des Weiteren mindestens ein zusätzliches Konditionierungsmittel umfasst.

11. Zusammensetzung nach dem vorhergehenden Anspruch, **dadurch gekennzeichnet, dass** das oder die zusätzlichen Konditionierungsmittel aus Poly-$\alpha$-olefinen, fluorierten Ölen, fluorierten Wachsen, fluorierten Gummen, Carbonsäureestern, Silikonen, kationischen Polymeren, mineralischen, pflanzlichen oder tierischen Ölen, Ceramiden, Pseudoceramiden und Mischungen davon ausgewählt sind.

12. Zusammensetzung nach einem der Ansprüche 10 oder 11, **dadurch gekennzeichnet, dass** das oder die zusätzlichen Konditionierungsmittel aus kationischen Polymeren, Silikonen und Mischungen davon ausgewählt sind.

13. Zusammensetzung nach dem vorhergehenden Anspruch, **dadurch gekennzeichnet, dass** das zusätzliche Konditionierungsmittel ein Silikon ist.

14. Zusammensetzung nach einem der Ansprüche 10 bis 12, **dadurch gekennzeichnet, dass** das Konditionierungsmittel ein kationisches Polymer ist.

15. Zusammensetzung nach einem der Ansprüche 10 bis 14, **dadurch gekennzeichnet, dass** die Konditionierungsmittel 0,001 bis 25 Gew.-%, vorzugsweise 0,005 bis 10 Gew.-%, bezogen auf das Gesamtgewicht der Zusammensetzung, ausmachen.

16. Zusammensetzung nach einem der vorhergehenden Ansprüche, **dadurch gekennzeichnet, dass** die Zusammensetzung des Weiteren mindestens ein Additiv umfasst, das aus Fettalkoholen mit 12 bis 26 Kohlenstoffatomen, anderen Polymeren als den erfindungsgemäßen Polymeren, Vitaminen, Duftstoffen, Perlglanzmitteln, Verdickungsmitteln, Geliermitteln, Spurenelementen, reizlindernden Mitteln, Sequestriermitteln, alkalisch oder sauer machenden Mitteln, Konservierungsstoffen, Sonnenschutzmitteln, Antioxidantien, Antihaarausfallmitteln, Antischuppenmitteln, Antifettmitteln, Treibmitteln sowie Mischungen davon ausgewählt ist.

**17.** Zusammensetzung nach einem der vorhergehenden Ansprüche, wobei das kosmetisch unbedenkliche Medium mindestens einen Bestandteil umfasst, der aus Wasser, hydrophilen organischen Lösungsmitteln wie Alkoholen, insbesondere linearen oder verzweigten C1-C6-Monoalkoholen und Polyolen und Etherglykolen ausgewählt ist.

**18.** Zusammensetzung nach einem der vorhergehenden Ansprüche, vorliegend in Form einer Haarbehandlungszusammensetzung, insbesondere für die Aufrechterhaltung der Frisur oder Formgebung von Haaren, zum Beispiel in Form von Shampoos, Gelen, Lotionen für Wasserwellen, Frisierlotionen, Zusammensetzungen zum Fixieren und Kämmen wie Lacke oder Sprays, Wash-out- oder Leave-in-Spülungen, Zusammensetzungen für Dauerwellen, Haarglättung, Färbung oder Bleichung oder auch in Form von Wash-out-Zusammensetzungen, die vor oder nach einer Färbung, Bleichung, Dauerwelle oder Haarglättung oder auch zwischen den beiden Schritten einer Dauerwelle oder einer Haarglättung aufzutragen sind.

**19.** Verwendung einer Zusammensetzung gemäß einem der Ansprüche 1 bis 18 zum Reinigen und/oder Entfernen von Make-up und/oder Konditionieren von Keratinmaterial.

**20.** Verwendung einer Zusammensetzung gemäß einem der Ansprüche 1 bis 18 als Shampoo für Keratinmaterial.

**21.** Kosmetisches Verfahren zur Behandlung von Keratinmaterial wie der Körper- oder Gesichtshaut, der Nägel, der Körperbehaarung, der Haare und/oder der Wimpern, **dadurch gekennzeichnet, dass** es darin besteht, eine kosmetische Zusammensetzung gemäß einem der Ansprüche 1 bis 18 auf das Keratinmaterial aufzutragen und im Anschluss gegebenenfalls eine Spülung anzuwenden, gegebenenfalls nach einer Wartezeit.

**22.** Verfahren zum Waschen von Keratinmaterial, insbesondere der Haare, **dadurch gekennzeichnet, dass** es darin besteht, eine Zusammensetzung gemäß einem der Ansprüche 1 bis 18 auf diese Materialien aufzubringen und dann gegebenenfalls mit Wasser zu spülen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9603969 A
- US 20060286057 A
- WO 200703784 A
- DE 102005014293
- US 2528378 A
- US 2781354 A


### Littérature non-brevet citée dans la description


- CTFA. 1982
- CTFA. 1993